# EUROPEAN PATENT APPLICATION

(11) **EP 4 312 230 A1**
(43) Date of publication of application: **31.01.2024**
(21) Application number: 23187558.4
(22) Date of filing: 25.07.2023
(51) Int. Cl.: G16H 50/30

(54) **METHOD FOR PROVIDING A CLINICAL DECISION SUPPORT, CORRESPONDING SYSTEM FOR PROVIDING A CLINICAL DECISION SUPPORT, AND COMPUTER PROGRAM PRODUCT**

(30) Priority: 25.07.2022 IT 202200015624
(71) Applicant: Aizoon S.r.l., 10146 Torino (TO) (IT)
(72) Inventor: MANGANARO, Lorenzo, 10146 Torino (TO) (IT); SABBATINI, Gianmarco, 10146 Torino (TO) (IT); BIANCO, Selene, 10146 Torino (TO) (IT); CIPOLLINI, Francesca, 10146 Torino (TO) (IT); COLOMBI, Davide, 10146 Torino (TO) (IT); VATTAKUNNEL, Shaji, 10146 Torino (TO) (IT); FALCO, Paolo, 10146 Torino (TO) (IT)
(74) Representative: Meindl, Tassilo

(57) **Abstract**

Described herein are solutions for providing a support to clinical decisions using a trained nonlinear classifier. For this purpose, a computer obtains (1202) for a patient (P) respective values (300) of a plurality of clinical data (p). Next, the computer generates a training dataset (306). For this purpose, the computer estimates (1204), by means of the nonlinear classifier, a respective class of risk (v) for the values of the patient (P) and adds the values of the patient (P) and the respective estimated class of risk (v) to the training dataset (306). The computer moreover generates (1406) a plurality of modified datasets (304) by modifying the values of the patient (P), estimates (1408) by means of the nonlinear classifier a respective class of risk (v) for each modified dataset (304), and adds the values of each modified dataset (304) and the respective estimated class of risk (v) to the training dataset (306).

Next, the computer determines a class of risk (v) from among the estimated classes of risk (v) that indicates a lower risk and trains (1112) a linear classifier configured to estimate the class of risk (v) as a function of the values of the clinical data (p) using the training dataset (306). The computer then determines a separation plane (402) of the linear classifier that separates the class of risk (v) estimated for the patient (P) from the class of risk (v) that indicates a lower risk, and uses the separation plane (402) to calculate the minimum modification required (MP) to the values of the patient (P) to modify the estimated class of risk (v) into the class of risk (v) that indicates a lower risk. Finally, the computer can provide support to clinical decisions by displaying the modification (MP) to the values of the patient (P) on a screen.

## Description

### Technical field

Various embodiments of the present disclosure regard solutions for providing clinical decision support, for example for providing a prescription to lower the class of risk for the patient associated to the presence of a given disease.

### Background

Various factors have contributed to the explosion of the amount of data that are available to physicians and can be used for treating a patient better. For instance, in addition to traditional clinical data (age, anamnesis, risk factors), the data coming from advanced imaging techniques and from molecular analysis can be taken into consideration. This enormous amount of data enables improvement of the options for evaluation and treatment of a patient, and consequently for prognosis, but also increases significantly the complexity of decision-making.

For instance, it is deemed that genetic profiles can be linked to the risk of developing a given illness and/or to the prognosis of the evolution of the illness, such as a particular type of cancer. In the case of cancer, where it has been possible to remove the lesion surgically, the prognosis is frequently quantified through a quantity that indicates the disease-free survival (DFS) time after a particular treatment.

With the invention and the recent reduction in cost of next-generation-sequencing (NGS) technology, there has progressively become available a large amount of omics data for bioinformatic analyses. In particular, NGS technology is a method of in-vitro analysis that comprises a sequencing in parallel and that enables sequencing of large genomes in a very short time. The term "omics" refers to data that identify, e.g., genomics, transcriptomics, proteomics, metabolomics, methylomics, radiomics, or metagenomics data.

This has in turn increased the interest in the development of machine-learning (ML) models that can decode the correlations between data, for example different genetic profiles (also referred to as "omics"), for example with reference to genetic mutation (differences between individuals encoded in different DNA sequences), to expression (differences between individuals and tissues encoded in different abundances of transcript, mRNA, for each gene), and to the number of copies (differences between individuals and tissues encoded by a different number of copies of a given gene). For example, in this context the European patents Nos. EP 1 977 237 B1, EP 2 392 678 B1, EP 2 836 837 B1, EP 3 237 638 B1 or EP 2 700 038 B1 may be cited.

For instance, to estimate the risk of developing a given illness or the disease-free survival time of a patient, the machine-learning model may comprise a parameterized mathematical function, such as an artificial neural network, configured to estimate a quantity of interest that corresponds, respectively, to the risk of developing the illness or to the disease-free survival time, as a function of the multi-source data, for example comprising omics data and other clinical data obtained for a given patient. In particular, by acquiring a training dataset that comprises the clinical data of a plurality of patients, and the respective information as to whether the patient has developed the illness or the respective disease-free survival time of each patient, a training algorithm can modify, typically through an iterative method, the parameters of the mathematical function, in such a way as to reduce the difference between the estimate of the quantity of interest and the respective data of the dataset. Consequently, once the learning model has been trained, the mathematical function can provide an estimate of the quantity of interest associated to a class of risk, i.e., the risk of developing the illness or the disease-free survival time, as a function of the respective clinical data (also comprising the omics data) of any patient. For instance, in this context, there may also be cited the Italian patent applications Nos. IT102022000001817 and IT102022000005861, the contents of which are incorporated herein for reference.

Consequently, these machine-learning models are able to estimate one or more variables of interest, the so-called endpoints, by analyzing a large amount of clinical data of other patients. For instance, the variable of interest may indicate:
- information on whether the patient has developed a given disease;
- the seriousness of the disease that the patient has developed, for example whether the patient has developed a mild form or a serious form (hospitalization and/or surgery in the hospital, and/or death of the patient on account of the disease); or
- disease-free survival time, for example data that indicate a period, for instance, a number of days, from the date of the surgical operation, such as a period that has elapsed up to a relapse or, in the absence of relapses, a period that has elapsed up to the last check or a period that has elapsed up to the death of the patient.

Such mathematical models are becoming increasingly complex (as in the case of deep learning) and are frequently considered black boxes. The relations between the clinical data received at input and the estimate of the variable of interest supplied at output are frequently extremely difficult to understand; this in turn reduces the confidence of the physician and of the patients in the models. This loss of confidence represents an obstacle to the diffusion of machine-learning models in clinical practice. In fact, the estimate provided by a predictive model applied to clinical practice can cause an action (or inaction) of the physician and, if it is wrong, may have serious consequences on the health of the patient. Consequently, it is fundamental for physicians to understand the reasons (i.e., the relation with the input data) on the basis of the response of a predictive model.

In this context, a further obstacle is represented by the high cost and complexity of integration of machine-learning models in tools that are useful for the physician, the so-called clinical-decision support systems (CDSSs). In fact, whereas machine-learning models are typically able to estimate/predict the prognosis of a given patient (for example, they can provide a class of risk for the patient or even an estimate of DFS), such models do not provide indications on the actions to be undertaken to improve prognosis.

In this respect document WO 2007/050186 A2 discloses a CDSS, wherein a processor obtains data records associated with input parameters and output parameters. For example, the input parameters may include information about parameters related to an individual patient's blood, urine, saliva and other fluid analysis, clinically measured information of individual patients, etc.

In a first step, the processor may select input parameters based on a mahalanobis distance between a normal data set and an abnormal data set of the data records. For example, the normal data set may include characteristic data associated with input parameters that produce desired output parameters. On the other hand, the abnormal data set may include any characteristic data that may be out of tolerance or may need to be avoided. Thus, essentially document WO 2007/050186 A2 is able to determine via the mahalanobis distance the input parameters, which have a strong correlation with the output parameters, mainly identifying a range for the selected input parameters which are correlated to a normal data set or an abnormal data set. For example, document WO 2007/050186 A2 mentions that a genetic algorithm may be used by the processor to search input parameters for the desired subset with the purpose of maximizing the mahalanobis distance.

In a second step, after having selected the input parameters, the processor may generate a medical risk stratification (MRS) process model, such as a neural network, to build interrelationships between input parameters and output parameters. Accordingly, once trained and validated, MRS process model may be used to predict values of output parameters when provided with values of input parameters.

In a third step, the processor identifies a desired distribution of input parameters such that a zeta statistic of the MRS process model is maximized or optimized. For example, an appropriate type of genetic algorithm may be used by the processor to search the desired distribution of input parameters with the purpose of maximizing the zeta statistic, also taking into account that statistical distributions of certain input parameters may be impossible or impractical to control.

Thus, the MRS model may be used to predict the risk score of individual data associated with a patient. Moreover, the processor may recalculate the risk score based on a change of the values of individual data. However, while document WO 2007/050186 A2 is able to determine a distribution of the input parameters associated with a normal risk score, document WO 2007/050186 A2 is unable to provide an individual prescription, corresponding to a local and minimum change to the individual data associated with a patient in order to reach a lower risk score.

### Summary

Various embodiments of the present disclosure hence provide new solutions for supplying prescriptions via a CDSS.

According to one or more embodiments, the above object is achieved through a method having the distinctive elements set forth specifically in the ensuing claims. The embodiments moreover regard a corresponding device, such as a corresponding computer program product that can be loaded into the memory of at least one computer and comprises portions of software code for implementing the steps of the method when the product is run on a computer. As used herein, reference to such a computer program product is intended as being equivalent to reference to a computer-readable means containing instructions for controlling a processing system in order to co-ordinate execution of the process. Reference to "at least one computer" is clearly intended to highlight the possibility of the present disclosure being implemented in a distributed/modular way.

The claims form an integral part of the technical teaching of the description provided herein.

As mentioned previously, various embodiments of the present disclosure regard solutions for providing a support to clinical decisions.

In particular, various embodiments of the present disclosure use a nonlinear classifier that has already been trained. The specific properties of this nonlinear classifier are not particularly relevant for the scope of the present disclosure, and it is sufficient that the nonlinear classifier is able to estimate the class of risk of a patient as a function of the values of the clinical data of the patient.

For instance, similar to the MRS process model of document WO 2007/050186 A2, to train the nonlinear classifier, a computer can obtain a training dataset comprising, for each of a plurality of reference patients, respective values of a plurality of clinical data and a respective value that identifies a class of risk. Then, the computer can train the nonlinear classifier using the training dataset.

However, the aforementioned nonlinear classifier is not able to provide prescriptions for reducing the class of risk of the patient. For this reason, document WO 2007/050186 A2 proposes the use of a genetic algorithm to search a desired distribution of input variables (associated with the normal data set). However, as mentioned before, in this way the genetic algorithm just obtains a global desired distribution of input variable and the solution of document WO 2007/050186 A2 is unable to provide an individual prescription corresponding to a local and minimum change to the individual data associated with a patient in order to reach a lower risk class.

Specifically, in various embodiments, to provide a prescription, the computer trains a further classifier, in particular a linear classifier. For this purpose, the computer obtains for a patient respective values of the clinical data. Next, the computer generates a new training dataset. For this purpose, the computer estimates by means of the nonlinear classifier a respective class of risk for the values of the patient, and adds the values of the patient and the respective estimated class of risk to the training dataset. Moreover, the computer generates a plurality of modified datasets by modifying the values of the clinical data of the patient. Next, the computer estimates, by means of the nonlinear classifier, a respective class of risk for the values of each modified dataset and adds the values of the modified datasets and the respective estimated classes of risk to the training dataset.

In various embodiments, the computer then determines a class of risk from among the classes of risk estimated for the modified datasets that indicates a risk lower than the class of risk estimated for the values of the patient, and trains a linear classifier configured to estimate the class of risk as a function of the values of the clinical data using the new training dataset.

Accordingly, compared to document WO 2007/050186 A2, the solutions disclosed herein evaluate local variations of the values of the clinical data of the patient in order to generate via the non-linear classifier a new training dataset, and an additional linear classifier is trained, which thus corresponds to a local model of the non-linear classifier in the vicinity of the values of the patient. In fact, since the additional classifier is linear, such an additional classifier may be used to determine the minimum variation to the values of the patient in order to reach a lower risk class.

Specifically, for this purpose, the computer determines a separation plane of the linear classifier that separates the class of risk estimated for the values of the clinical data of the patient from the class of risk that indicates a lower risk. For instance, in various embodiments, the class of risk that indicates a lower risk corresponds to the class of risk from among the classes of risk estimated for the modified datasets that indicates a minimum risk. In this case, the linear classifier may be a multi-class classifier, and the separation plane of the linear classifier separates the class of risk that indicates a minimum risk from all the other classes of risk estimated for the modified datasets.

Alternatively, the computer can replace the class of risk estimated for the values of the patient with a first value and associate a second value to the class of risk that indicates a minimum risk. Then, the computer can replace each class of risk estimated for a respective modified dataset with the first value if the respective class of risk indicates a risk higher than the class of risk that indicates a minimum risk and the second value if the respective class of risk corresponds to the class of risk that indicates a minimum risk. In this case, the computer can then train a binary linear classifier configured for classifying the values of the clinical data with the first value or the second value, where the separation plane of the linear classifier separates the class of risk that indicates a minimum risk from all the other classes of risk estimated for the modified datasets.

In various embodiments, the computer then uses the separation plane to calculate the minimum modification required to the values of the clinical data of the patient to change the class of risk estimated for the patient into the class of risk that indicates a lower risk. For instance, for this purpose, the computer can determine the clinical data to be modified by calculating the normal to the separation plane. Moreover, the computer can determine the degree of modifications to the clinical data to be modified by calculating the minimum distance between the values of the clinical data of the patient and the separation plane.

Accordingly, in document WO 2007/050186 A2, the computer may only display a desired distribution of input variables and evaluate via the MRS model specific changes to the values of the clinical data of the patient. Conversely, in the solutions disclosed herein, the computer can provide a support to clinical decisions by displaying the aforesaid minimum modifications required to the values of the clinical data of the patient on a screen.

In various embodiments, the computer also takes into consideration one or more constraints for the modifications to the values of the clinical data of the patient. In general, in this case, the computer can generate the plurality of modified datasets by modifying the values of the patient as a function of the constraints, and/or the computer can verify whether the minimum modification required to the values of the patient satisfies the constraints. For instance, these constraints may comprise at least one of the following: a blacklist containing clinical data that cannot be modified; a list containing the definition of the range of values that are acceptable for one or more of the clinical data; and/or data that specify one or more correlation matrices, where each correlation matrix specifies the correlation between a plurality of clinical data.

In various embodiments, the constraints may comprise a list of actions, such as the taking of a given drug, a given medical intervention, and/or a given physical activity, where each action specifies a modification to one or more of the clinical data. In this case, the computer can then determine whether one of the actions is compatible with the minimum modification required by verifying whether the direction of the modifications to the one or more clinical data corresponds substantially to the direction and possibly the degree of the minimum modification required. Consequently, in the case where one of the actions is compatible with the minimum modification required, the computer can display the action, i.e., the prescription, on the screen.

### Brief description of the drawings

The embodiments of the present disclosure will now be described with reference to the annexed drawings, which are provided purely by way of non-limiting example and in which:
- Figure 1 shows a flowchart of a method and corresponding operation of a computer configured to estimate a variable of interest as a function of the clinical data of a patient via a learning step and an estimation step;
- Figure 2 shows an example of a processing system that is able to implement the operation of Figure 1;
- Figure 3 shows an embodiment of the learning step of Figure 1;
- Figures 4, 5, and 6 show details of the learning step of Figure 3;
- Figure 7 shows an embodiment of the estimation step of Figure 1;
- Figure 8 shows a detail of the estimation step of Figure 7;
- Figure 9 shows a flowchart of an embodiment of operation of a system for providing support to clinical decisions according to the present disclosure; and
- Figures 10, 11 and 12 show details of operation of the embodiment of Figure 9.

### Detailed description

In the ensuing description numerous specific details are provided in order to enable an in-depth understanding of the embodiments. The embodiments may be implemented without one or various specific details, or with other methods, components, materials, etc. In other cases, well-known operations, materials, or structures are not represented or described in detail so that the aspects of the embodiments will not be obscured.

Reference throughout the ensuing description to "an embodiment" or "one embodiment" means that a particular characteristic, distinctive element, or structure described with reference to the embodiment is comprised in at least one embodiment. Thus, use of phrases such as "in an embodiment" or "in one embodiment" in various points of this description do not necessarily refer to one and the same embodiment. Moreover, the details, characteristics, distinctive elements, or structures may be combined in any way in one or more embodiments.

The references used herein are provided merely for convenience and do not define the scope or meaning of the embodiments.

As explained previously, various embodiments of the present disclosure provide solutions for explaining the behaviour of a given machine-learning model. In fact, this not only enables clarification of the relation between the estimate of the variable of interest provided by the machine-learning model and the clinical data (also comprising the omics data) received at input, but can also be used for providing a support to clinical decisions, for example to provide advice on which treatment should be adopted to improve the estimate of the variable of interest.

For instance, as explained previously, to provide a more accurate prognosis, lately clinical data have been taken into consideration, increasingly often comprising omics data, where the entire set of the data is processed by means of a machine-learning algorithm. For instance, in various embodiments, the machine-learning model is used to estimate the disease-free survival time for patients affected by non-small cell lung cancer (NSCLC), who have undergone a surgical operation and have obtained complete removal of the neoplasm. However, the machine-learning model could also estimate the disease-free survival time for other illnesses, and in particular for other types of cancer. For instance, such an estimate may represent auxiliary information fundamental for oncologists, who can make better-informed decisions, for example vary the frequency of the follow-up checks as a function of the time estimated, for instance increasing the frequency of the checks for patients with an estimated time that is short. In general, the machine-learning model could also estimate one or more other quantities of interest that are correlated with the clinical data (also comprising the omics data) of a patient, such as the risk that a patient will develop a given illness and/or the risk that a patient will develop a serious form of the illness.

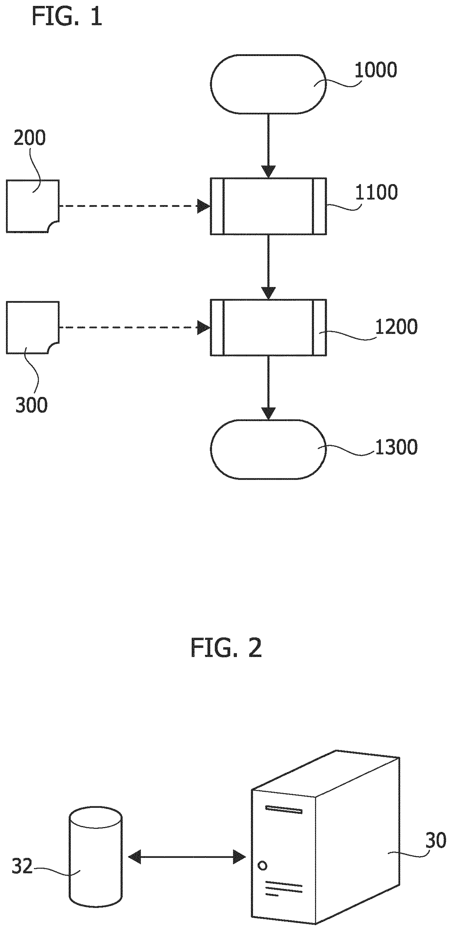

Figure 1 shows an embodiment of operation of a computer 30 configured for estimating a variable of interest for a given patient (output datum) as a function of respective values of a set of variables (input data).

For instance, as illustrated in Figure 2, the computer 30 may be implemented by any processing system, possibly also in distributed form, and may comprise, for example, a personal computer, a smartphone or a tablet, and/or a remote server. Consequently, operation of the computer 30 can be implemented via software code run on one or more processors.

In the embodiment considered, after a start step 1000, the computer 30 trains, in a step 1100, a machine-learning algorithm using a training dataset 202 that comprises clinical data for a plurality of reference patients PR. For instance, the training dataset 202 can be stored in one or more databases 32 managed by the computer 30. Consequently, in a step 1200, the computer 30 can use the trained algorithm to estimate the quantity of interest, for example the disease-free survival time of a patient as a function of the respective clinical data 300, and the process terminates in an end step 1300.

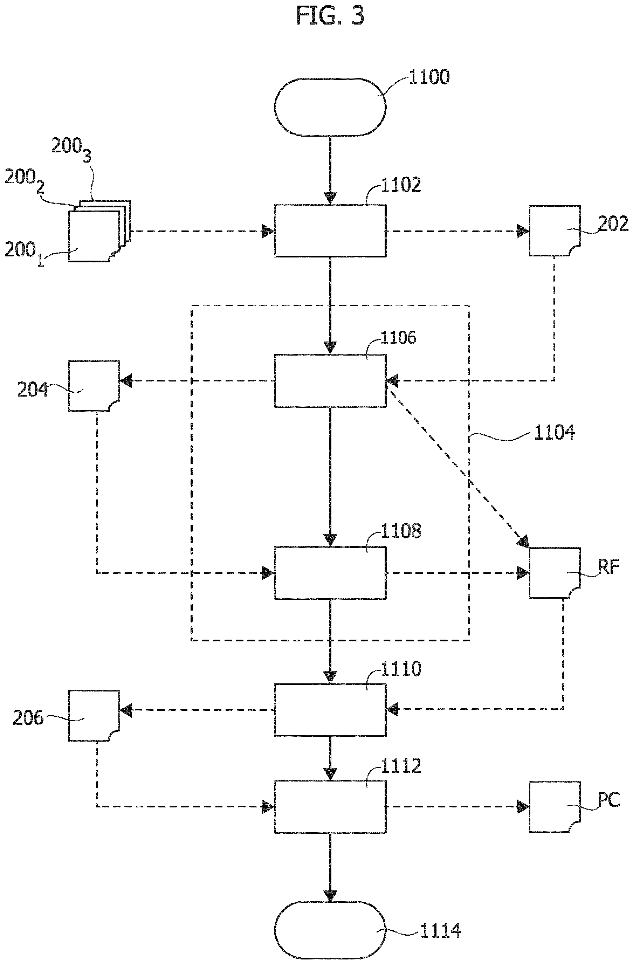

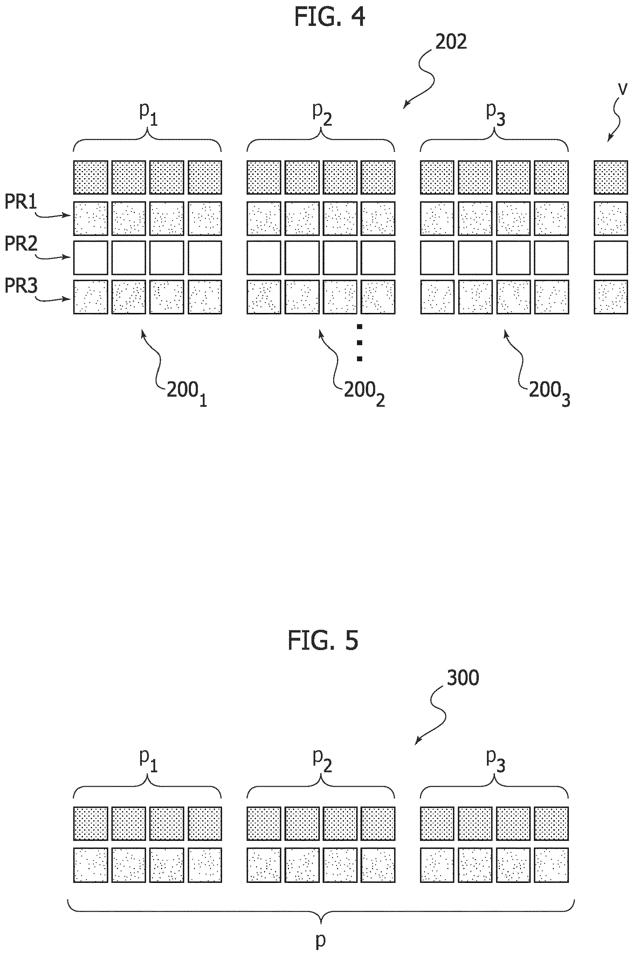

Figure 3 shows a possible embodiment of the learning/training step 1100. Once the learning step 1100 has been started, the computer 30 obtains, in a step 1102, a training dataset 202. For instance, as also shown in Figure 4, the computer 30 may receive, in step 1102, one or more datasets of clinical data 200, for example datasets 200₁, 200₂ and 200₃, and generate a single dataset 202. In various embodiments, each dataset 200 corresponds to a table, list, or data matrix that comprises the values for a respective number of variables, for example variables p₁, p₂, and p₃, for a plurality of reference patients PR. Consequently, the computer 30 can remove, in step 1102, possible variables that are repeated in the datasets 200, remove reference patients PR for whom not all the data are available, etc. Hence, in general the dataset 202 comprises p variables of clinical data for m reference patients PR.

For instance, a first dataset 200₁ may comprise omics data, for example NGS (RNA-seq) transcriptomics data. In particular, with reference to NGS data, each variable corresponds to the gene expression of a particular gene. For instance, the dataset 200₁ may correspond to a data matrix, where each row of the matrix represents a reference patient PR (sample), for example patients PR1, PR2, PR3, etc., and each column represents the gene expression of a particular gene, for example expressed in transcripts per million (TPM). Likewise, the dataset 200₂ may comprise data of variation of the number of copies. In this case, the dataset 200₂ corresponds to a table, list, or data matrix that comprises the values for a number p₂ of variables, where each variable corresponds to the variation of the number of copies of a particular gene. Finally, the dataset 200₃ may comprise mutation data. In this case, the dataset 200₃ corresponds to a table, list, or data matrix that comprises the values for a number p₃ of variables, where each variable corresponds to the mutation data of a particular gene. For instance, some examples of these data are made available by *The Cancer Genome Atlas* (TCGA) and may be freely downloaded from many sources, such as https://gdac.broadinstitute.org/ or https://www.cbioportal.org/. For instance, with reference to NSCLC, a dataset 200 may be used that comprises the lung-adenocarcinoma (LUAD) TCGA databases and/or the lung-squamous-cell-carcinoma (LUSC) TCGA databases, which currently represent the main histological subtypes of NSCLC.

In general, in addition to or instead of the omics data (including transcriptomics, genomics, epigenomics, metabolomics, methylomics, and/or proteomics data), the dataset 202 may also comprise other datasets 200, for example one or more datasets 200 obtained via laboratory analyses, e.g., blood tests, radiological data (e.g., x-ray, CT, MR, PET), and/or medical and anamnestic data in general (e.g., age, sex, history of smoking, etc.). For example, reference can be made to WO 2007/050186 A1 for other possible datasets 200. Moreover, the dataset 202 comprises, for each reference patient PR, the value of the variable of interest v, such as:
- information on whether the patient has developed a given disease;
- data that indicate the seriousness of the disease that the patient has developed, for example whether the patient has developed a mild form or a serious form (hospitalization and/or surgery in the hospital, and/or death of the patient on account of the disease);
- data that identify the disease-free survival time, for example data that indicate a period, for instance, a number of days, from the date of the surgical operation, such as a period that has elapsed up to a relapse or, in the absence of relapses, a period that has elapsed up to the last check or a period that has elapsed up to the death of the patient; or
- data that identify other information regarding the course of the disease that is relevant for the patient and for the physician, such as the stay in hospital envisaged, and the risk of death or of other clinically relevant events, such as onset of complications or the likelihood of adverse events in the case of surgical operation.

In various embodiments, the variable v can then be associated to a class of risk, and the learning method is a classifier configured for dividing the space of the variables p into regions in such a way as to estimate the variable of interest v as a function of the variables p.

Step 1102 is purely optional since the computer 30 could receive directly a similar dataset 202. In fact, it is sufficient for the computer 30 to obtain, in step 1102, a dataset 202 that comprises, for each reference patient PR, the values of a plurality of variables p of clinical data and a respective value of the variable of interest v. As illustrated in Figure 5, in the embodiment considered, the data 300 of a patient P thus comprise the data of the variables p, for example, with p'₁ representing the mRNA gene expressions 300₁, p'₂ representing the variations of the number of copies 300₂, and p'₃ representing the mutations 300₃. Consequently, the computer 30 should estimate, in step 1200, a respective value v as a function of the above data 300.

Considering the high number of clinical data, the aforesaid training dataset 202 is typically analyzed in a step 1104 that implements one or more feature-extraction or feature-selection algorithms. Such algorithms have in common the fact that the computer 30 stores, in step 1104, one or more mapping rules RF used for generating a plurality of features F as a function of the variables p of the training dataset 202.

For instance, Figures 3 schematically illustrates a feature-extraction step 1106 and a feature-selection step 1108.

For instance, the matrix 202 frequently comprises a number of variables p (for example, the columns) much higher than the number m of reference patients PR (for example, the rows), with m « p. From a mathematical standpoint, the matrix is hence written in a redundant form since it can be easily demonstrated that the rank of the columns of the matrix is equal to the rank of the rows. For instance, in various embodiments, the computer 30 can generate, in step 1106, a reduced matrix 204 by projecting the matrix 202 in an m-dimensional subspace, for example via a principal-component analysis (PCA). PCA and its variants are well known to the person skilled in the branch. For instance, for this purpose, it is possible to cite the book by T. Jolliffe, "Principal Component Analysis", Springer Series in Statistics, Springer-Verlag, New York, 2002, ISBN 0-387-95442-2, the contents of which are incorporated for this purpose herein for reference. The person skilled in the branch will appreciate that numerous other feature-extraction methods are known, and there may be cited, for example, the webpage "Feature extraction" of Wikipedia^{®}, available for example at the link https://en.wikipedia.org/wiki/Feature_extraction, the contents of which are incorporated herein for reference.

Consequently, in the embodiment considered, the computer 30 can select, in step 1108, a sub-set of the variables of the matrix 204 (or alternatively directly of the matrix 202). The person skilled in the branch will appreciate that there are also known numerous feature-selection methods, and for example the webpage "Feature selection" of Wikipedia^{®} may be cited, available for example at the link https://en.wikipedia.org/wiki/Feature_selection, the contents of which are incorporated herein for reference. For instance, with reference to the specific application, the inventors have noted that a LASSO model is particularly useful. This method is well known to the person skilled in the branch and described, for example, in the article by Tibshirani, Robert, "Regression Shrinkage and Selection via the Lasso", Journal of the Royal Statistical Society, Series B (methodological), 1996, Wiley, 58 (1): 267-88, DOI:10.1111/J.2517-6161.1996.TB02080.X, the contents of which are incorporated herein for reference. For instance, by training a LASSO model in step 1108, the computer 30 can generate the list RF by removing all the variables of the matrix 204 (or directly of the matrix) the LASSO coefficients of which are equal to 0. However, the LASSO method can be replaced with other feature-selection methods, for example one or more methods chosen from the following list:
- wrapper methods, for example using a recursive feature elimination, a forward feature selection, or a backward feature selection;
- filters, for example based upon the chi-squared method, Pearson correlation, relief or Fisher score; or
- embedded methods, for example based upon decision trees, the so-called random forests, sparse multinomial logistic regression, automatic relevance determination, or regularization-based methods, for example ridge and elastic net.

For instance, for this purpose it is possible to cite the article by A. Jović, *et al., "A review of feature selection methods with applications",* May 2015, 38th International Convention on Information and Communication Technology, Electronics and Microelectronics (MIPRO), DOI:10.1109/MIPRO.2015.7160458, the contents of which are incorporated herein for reference. Moreover, the document 102022000005861 describes a solution in which the mapping rules RF are generated on the basis of an analysis of a multi-layer network.

Consequently, the feature-selection step 1008 selects only the variables p that have a correlation with the variable of interest v. Instead, the feature-extraction step transforms the input variables p into new variables/features (for example, by making linear combinations thereof). Consequently, feature extraction by its very nature jeopardizes the interpretability of the model since the input variables p tend to have an understandable meaning, whereas the new variables do not have an immediate practical meaning. For this reason, in various embodiments, the computer 30 omits the feature-extraction step 1006 and uses only a feature-selection step 1008.

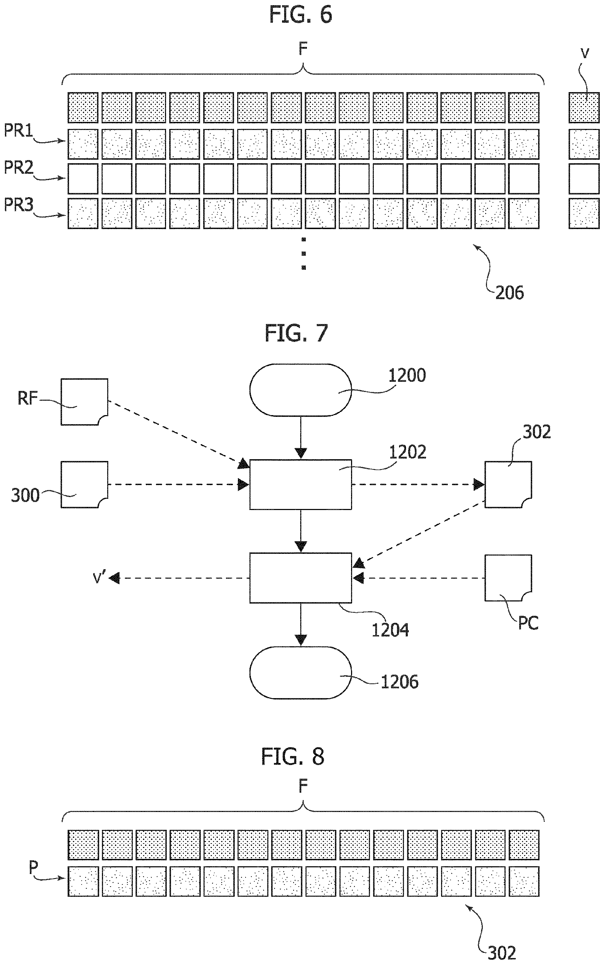

Consequently, in the embodiment considered, the mapping rules RF may comprise the rules used in steps 1106 and/or 1108, respectively, to extract and/or select the features F. Hence, in a step 1110, the computer 30 can generate a training dataset 206 using the mapping rules RF to calculate for each reference patient PR the values of the features F as a function of the respective data 202 of the reference patient PR (see Figure 6). For instance, the value of the first feature of each reference patient PR is calculated as a function of the data 200 of the respective reference patient PR using the first mapping rule RF.

Consequently, in a step 1112, the computer 30 can use the training dataset 206 (or the dataset 204 or directly the dataset 202) for training a classifier, such as a machine-learning algorithm, that is able to estimate the value of the variable of interest v as a function of the values of a given set of features F. For instance, the classifier may be an artificial neural network, a support-vector machine, or any other parameterized mathematical function configured to estimate the variable of interest v as a function of the values of the features F. In this case, the computer 30 can vary, in step 1112, the values of the parameters PC of the mathematical function in such a way as to minimize a cost function calculated on the basis of the differences between the estimates v' of the variable v supplied by the mathematical function and the value of the variable v in the dataset 206. Finally, the training procedure 1100 terminates in an end step 1114. For instance, in this context, the Italian patent application No. 102022000001817 describes a machine-learning method that is able to estimate the disease-free survival time of a patient.

Figure 7 shows an embodiment of step 1200. In particular, when step 1200 has been started, the computer 30 obtains the data 300 of the patient P and uses the mapping rules RF to calculate the values 302 of the features F as a function of the data 300 of the patient P (see also Figure 8). Next, the computer 30 uses the trained classifier, as identified, for example, via the values PC of the parameters of the mathematical function, to supply the estimate v' of the variable of interest v as a function of the values 302 of the features F determined for the patient. Finally, the procedure terminates in an end step 1206.

Consequently, in various embodiments, the learning method receives a dataset 202 of data that may comprise omics data (for example, transcriptomics, genomics, epigenomics, methylomics, and/or proteomics data), data obtained via laboratory analyses (for example, blood tests, radiological data), general clinical data (for example, age, sex, history of smoking, etc.), or a combination of these data. The learning method consequently supplies at output a variable of interest that typically indicates a risk index. For this purpose, the learning model may supply directly the value of a class, for example in the case where the learning model is implemented with a support-vector machine or a k-means estimate. However, the learning model may also comprise one or more machine-learning algorithms that supply estimates of one or more continuous values, for example by means of one or more neural networks. In this case, the learning model may also comprise a decision module, i.e., a classifier, which generates a class of risk as a function of the estimates of the one or more continuous values. For instance, in the simplest case, the decision module can discretize the continuous value. Consequently, in general, the learning method may correspond to any classifier that is able to divide the space of the features F (or with reference to the entire step 1100, the space of the variables p) into regions, so as to be able to estimate a variable of interest v that indicates a class of the patient P on the basis of the values of the features F (or with reference to the entire step 1100 to the values of the variables p) obtained for the patient P.

As explained previously, these learning methods/classifiers of clinical data 202 are becoming increasingly complex and are frequently considered black boxes; i.e., the relations between the clinical data 202 received at input and the estimate v' of the variable of interest v supplied at output are frequently extremely difficult to understand. This also renders integration of the machine-learning models in the CDSSs particularly difficult because these models do not provide indications on the actions to be undertaken to improve the estimate v' of the variable of interest v, for example to move the patient P from a high-risk class into a lower-risk class, for example a class with a higher DFS value.

In fact, for this purpose the black-box model should be implemented "backwards" in the CDSS. Starting from the knowledge of the domain (available drugs, possible interventions, etc.), the CDSS should thus implement a model based upon rules that approximate the results of the black-box model to calculate which are the best results possible of a particular intervention for a patient. This approach, however, presents various limits and is extremely costly. In fact, such an approach requires a profound knowledge of the domain of the available options and requires constant updating, for example when a new therapy becomes available. Moreover, since the CDSS model is intrinsically an approximate re-implementation of the original model, this model requires separate re-validations, with each iteration of the CDSS that could require a validation step in the form of clinical trial.

As mentioned previously, various embodiments of the present disclosure provide solutions that are able to decode in a way understandable for humans the black-box model of machine learning, passing from a black-box model to a white-box model. In fact, in this way, a CDSS is able to generate prescriptions without knowing the clinical domain, using as input for its development exclusively a validated black-box model. This type of CDSS will improve transparency and reduce the costs of implementation of personalized prescriptions in clinical practice, thus filling the gap between modelling and practice, creating a new standard for precision medicine.

As explained previously, step 1100 trains a risk classifier that receives a set 202 of different clinical and omics variables p to estimate a class of risk v, for example the risk of relapse of a pathology (for instance, cancer) within a given number of years (DFS). As explained previously, considering the high number of variables taken into consideration, this classifier is typically not readily understandable. In fact, the classifier provides an evaluation of the endpoint (the class of risk). However, this model typically provides a poor visibility on the reasons for which the model has made the evaluation, for example because the machine-learning model uses, in step 1112, one or more nonlinear mathematical functions; i.e., the machine-learning model combines the different variables p in a nonlinear way.

In various embodiments, the present disclosure hence provides solutions for determining a new model that approximates the original risk classifier. In this context, the inventors have noted that there has recently been proposed a new paradigm known as "explainable AI" (XAI), which comprises methods (referred to as "explainers") that try to understand why an algorithm, given a certain input, yields a given response. These solutions thus enable development of metrics and tools that provide a decoding that is understandable to humans of the black boxes of machine learning, passing from a black-box model to a white-box model.

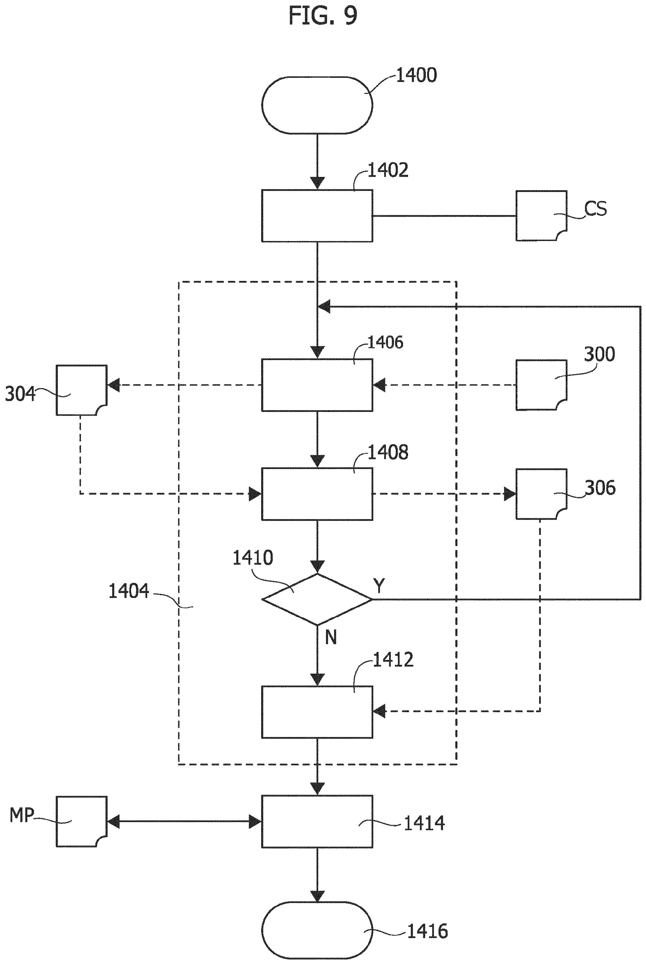

Figure 9 shows a possible embodiment of operation of a CDSS that can be implemented via the computer 30 or a further computer.

In particular, after a start step 1400, which is started, for example after step 1300, the computer generates, in a step 1404, a new model for the patient P. In particular, given a trained model that predicts a class v for the data 300 of a patient P (for example, a "high risk" of a specific adverse event such as recurrence of cancer, death, side effects, failure of a treatment), the computer is configured for analyzing, in a step 1404, the behaviour of the model trained in step 1100.

In particular, in various embodiments, the computer 30 generates for this purpose, in a step 1406, a dataset 304 by modifying the value of a variable p or the values of a plurality of variables of the dataset 300 of the patient P. Next, the computer supplies, in a step 1408, the modified dataset 304 at input to the machine-learning model and obtains the estimated value v supplied by the machine-learning model for that modified dataset 304. As schematically illustrated via a verification step 1410, the computer repeats steps 1406 and 1408 for a plurality of times. For instance, in the case where the computer should make further modifications of the dataset 300 (output "Y" from the verification step 1410), the computer can return to step 1406.

Consequently, in the case where the computer has made all the modifications of the dataset 300 (output "N" from the verification step 1410), the computer can determine, in a step 1412, the impact of the variables p on the estimate v. In fact, given the estimates of the variables v for the modified datasets 304 of a specific patient P, the computer is able to understand which input variables p are determining for the estimate v. For instance, in this way, the computer could detect that the risk of a patient having a heart attack is high because a value of cholesterol is high, which indicates the fact that the patient has hypercholesterolaemia.

Finally, in various embodiments, the computer can generate, in a step 1414, a specific set of modifications MP to the data 300 that should be made to reduce the estimate v of the risk, for example by changing the result of the estimate v from a "high risk" class to a to a "low risk" class.

Operation of step 1404, which is configured for generating a new model (being substantially a local model around the values of the dataset 300 of a patient P) on the basis of the behaviour of a black-box model obtained with machine learning, is in itself well known in the art. In fact, there exist various algorithms in the literature for these explainers. For instance, the best known in the literature are SHAP (SHapley Additive exPlanations) and LIME (Local Interpretable Model-agnostic Explanations). For instance, SHAP and LIME are implemented in Python software libraries and are compared in the scientific paper by Jesse He and Subhasish Mazumdar, "Comparing LIME and SHAP using Synthetic Polygonal Data Clusters", International Journal for Infonomics (IJI), Volume 14, Issue 1, 2021.

As explained with reference to Figure 9, during step 1404 (i.e., the explainer), the computer receives a point of the dataset, which in the present solution corresponds to the dataset 300 of a patient P, of which it is desired to know the explanation. Next, the computer creates, via step 1406, a plurality of modified datasets 304 that represent synthetic points that are close to the point to be explained, i.e., the dataset 300. The way in which this closeness of synthetic points is created depends upon the type of explainer that is used. As explained previously, in general, step 1406 can generate the modified datasets 304, i.e., the synthetic points, by modifying single values and/or a plurality of values of the dataset 300.

For example, in various embodiments, the computer determines at step 1406 for each input variable p a respective probability distribution. For example, in various embodiments, the probability distribution of each variable p corresponds to a respective gaussian/normal distribution with a respective standard deviation centered on the respective value of the variable p for the patient P. For example, the standard deviation of a given variable p may be predetermined and may correspond, e.g., to the standard deviation of the values of the dataset 202 for the respective variable p. Accordingly, in this way the computer 30 may generate, as in a Monte Carlo simulation, a synthetic point by extracting randomly (drawing) for each variable p a respective value according to the respective probability distribution.

Each synthetic point 304 is then supplied, in step 1408, to the trained model, and the respective estimated value v is associated to the respective synthetic point/dataset 304. In general, the trained model can then be stored in the computer itself, or the computer can exchange the information with the computer 30.

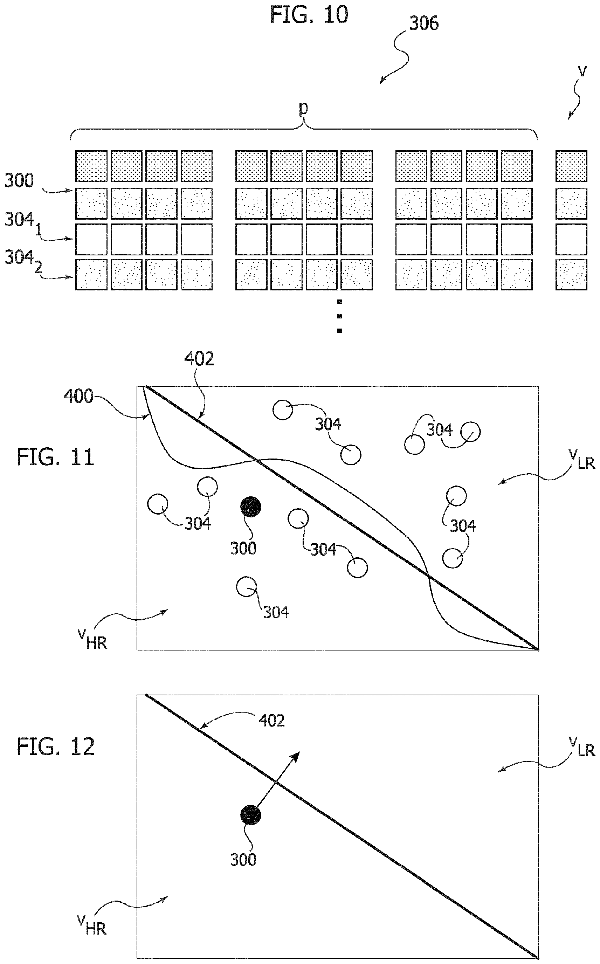

Consequently, as illustrated in Figure 10, step 1408 can generate a dataset 306, for example in the form of a matrix, list, or tables, which comprises a sequence of synthetic points/modified datasets 304, for example datasets 304₁, 304₂, etc., and, for each synthetic point/dataset 304, a respective value of the variable of interest v estimated by the machine-learning model for the respective synthetic point/dataset 304. Preferably, the dataset 306 also comprises the dataset 300 with the respective value v.

Finally, the computer generates, in step 1412, a new model on the basis of the dataset 306. In particular, the dataset 306 basically corresponds to a new training dataset. Consequently, in various embodiments, the computer can train, in step 1412, a new classifier. In particular, in various embodiments, whereas the classifier trained in step 1100 is a nonlinear classifier, the classifier trained in step 1412 is a linear classifier. Consequently, in various embodiments, the computer 30 uses the training dataset 306 for training a linear classifier that is able to estimate the value of the variable/class of interest v as a function of the values of the set of variables p. Linear classifiers are in themselves well known in the art, and reference may be made, for example, to the corresponding webpage "*Linear classifier*" of Wikipedia^{®}, available for example at the link https://en.wikipedia.org/wiki/Linear_classifier, the contents of which are incorporated herein for reference. Basically, for this purpose, the computer 30 determines, in step 1414, the weights of the linear classifier.

In this context, Figure 11 shows an example of a classification for the exemplary case of just two variables p. In particular, when, in step 1100, a given dataset 300 or a given modified dataset 304 is supplied at input to the trained learning model, the nonlinear classifier uses a nonlinear separation 400 to divide the points 300 and 304 into a first group that has a first value v_{LR} for the value of the variable v, for example indicating a low risk, and a second group that has a second value v_{HR}, for example indicating a high risk. Instead, the computer trains, in step 1412, a linear classifier. Consequently, when a given dataset 300 or a given modified dataset 304 is supplied at input to the linear classifier, the latter uses a linear separation 402 to divide the points 300 and 304 into the first group with first value v_{LR} and the second group with second value v_{HR}.

Consequently, step 1404 provides a local explanation, i.e., an approximation of the behaviour of the black-box model in the vicinity of the point of interest, i.e., of the original dataset 300 of the patient P.

As mentioned previously, in various embodiments, once the linear classifier has been trained, the computer uses, in step 1414, the aforesaid classifier to issue a prescription. In particular, as illustrated in Figure 12, the computer 30 is configured for proposing modifications to the values of the variables p in such a way as to move the point 300 from its own class v into a class v with a lower risk, for example, to move the point 300 from the class v_{HR} to the class v_{LR}. For instance, with reference to the DFS value, the class with lower risk may correspond to a higher DFS value.

In this context, the inventors have noted that the weights of the linear classifier indicate for each parameter p the importance of the parameter p for determination of the variable of interest v. In particular, the parameters identified by the linear classifier (represented in Figure 12 by the slope of the straight line 402, which represents the local approximation of the nonlinear classifier around the point 300), make it possible to trace the direction perpendicular to the straight line 402. This direction identifies the most efficient movement (in so far as it is the shortest) to move the point 300 from the class v_{HR} to the class v_{LR}. Such a movement assumes concrete form in a prescription of variation of the input variables (i.e., in the case provided by way of example of Figure 12, in the variation of the two dimensions x and y necessary for the point 300 to pass beyond the straight line 402).

In particular, as explained previously, step 1414 should propose modifications to the values of the patient P for the parameters p to move the class of risk of the patient into a lower-risk class. In this context, on the basis of the data of the dataset 306, the linear classifier is already trained by taking into consideration only classes of risk that can be obtained by modifying the original dataset 300.

Consequently, in various embodiments, the computer determines, in step 1414, the class of risk v associated to the data 300 of the patient P, i.e., the value of the variable of interest v estimated by the nonlinear classifier. Moreover, in various embodiments, the computer is able to determine the order of the classes of risk. For instance, for this purpose, the computer can store a list of possible values of classes of risk that are ordered on the basis of the corresponding risk, or the class of risk may correspond to a numeric value, and the risk associated to the class increases (or alternatively decreases) with the respective value of the class.

In particular, in various embodiments, the linear classifier is a binary classifier. In this case, in a first embodiment, prior to training of the linear classifier, the computer replaces, in step 1412, the value of the class of risk v obtained for the data 300 of the patient P with a first value and replaces each class of risk v obtained for a respective dataset 304 with:
- the first value if the respective class v corresponds to the class v obtained for the data 300 or indicates a class with a higher risk; and
- a second value if the respective class v indicates a class with a lower risk.

Consequently, in this case, the computer trains, in step 1414, a binary classifier that comprises only a single (multi-dimensional) separation plane 402 (see also Figure 11 for the exemplary case of a line of separation 402 for two variables p), which hence classifies a given point with the first value or the second value. Linear classifiers and the respective separating hyperplane(s) are *per se* well-known in the art. For example, a well-know linear classifier is the perceptron.

In this case, the computer may determine the prescription, i.e., the modifications MP to the data 300, in such a way as to obtain the second value (associated to the classes with lower risk), where the prescription comprises two items of information:
- the variables that have to be modified, i.e., the direction in which to move in order to shift the point 300 beyond the multi-dimensional separation plane 402 with the shortest path; and
- the degree of the modifications to these variables.

In particular, considering that the classifier uses a separation plane 402, the shortest vector that shifts the point 300 beyond the separation plane 402, i.e., into the second class, corresponds to a vector that passes through the point 300 and is perpendicular to the separation plane 402, where the length of the vector corresponds to the distance between the point 300 and the separation plane 402. For instance, for this purpose, the computer can determine the direction of the vector MP by calculating the normal to the multi-dimensional separation plane 402. In particular, since it is a plane, this calculation is easy as compared to a nonlinear separation because the normal has the same direction in each point of the plane 402. Moreover, knowing the normal, the computer can determine the (minimum) distance between the point 300 and the plane 402. Consequently, knowing the direction and the distance, the computer can determine the prescription/ modifications MP as vector (with minimum length) for shifting the point 300 beyond the plane 402.

The above embodiment presents the drawback that the prescription does not distinguish between the different classes of risk that can be reached by the point 300. Consequently, in a second embodiment, the linear classifier is a multi-class classifier. In this case, the trained linear classifier comprises a plurality of planes of separation 602. For instance, in a typical multi-class linear classifier, each class is separated from the other classes via a respective separation plane 402. Consequently, in a multi-class classifier, the computer can calculate, for each class that indicates a lower risk, a respective prescription/respective modifications MP that correspond to the respective vector (with minimum length) for shifting the point 300 beyond the respective separation plane 402.

In various embodiments, instead of determining a respective prescription for each class with lower risk, the computer can determine only the prescription/modifications MP that correspond to the vector (with minimum length) for shifting the point 300 beyond the separation plane 402 associated to the class with lowest obtainable risk. As explained previously, this class does not necessarily correspond to the class with lowest risk in absolute terms, but is represented by the class with the lowest risk obtainable for the modified datasets 304.

Consequently, to emulate this behaviour of a multi-class classifier, in a third embodiment, the computer determines, in step 1412, the value of the class of the dataset 304 with the lowest risk, replaces the value of the class of risk v obtained for the data 300 of the patient P with a first value and the value of the class with the lowest obtainable risk with a second value, and replaces each class of risk v obtained for a respective dataset 304 with:
- the first value if the respective class v indicates a class of risk with risk greater than the class with the lowest obtainable risk; and
- the second value if the respective class v corresponds to the class with the lowest obtainable risk.

Consequently, in this way the computer can train a binary linear classifier, which hence comprises a single separation plane 402, and determine the prescription MP as the shortest vector that moves the point 300 into the second class (with the lowest obtainable risk for the patient). As mentioned previously, for this purpose the computer can determine the direction of the vector MP by calculating the normal to the multi-dimensional separation plane 402, and then calculate the (minimum) distance between the point 300 and the plane 402.

In various embodiments, the computer can also verify whether the lowest-risk class thus determined comprises at least one given number of points 304, thus excluding the possibility of the respective points 304 representing outliers that in actual fact cannot be reached, i.e., the computer can determine the class with the lowest obtainable risk as the class with lowest risk that comprises at least one given number of points/datasets 304.

Consequently, in general, at the end of step 1414, the computer has determined a vector MP that indicates the modifications to be made to the data 300 in such a way as to shift the point into a class with lower risk, and preferably into the class with the lowest obtainable risk. Consequently, in step 1414, the computer can display a prescription on a screen of the computer, where this prescription comprises the modifications to the values of the variables p, i.e., the data of the vector MP. In general, instead of showing all the modifications, the computer may also display just the main modifications.

In this context, the inventors have noted that some modifications to the data 300 are not feasible or reasonably feasible. Consequently, in various embodiments, the computer uses, in a step 1402, constraints CS. In general, these constraints CS can be used in the step of configuration of the CDSS (for example, to choose the parameters of operation of the computer, such as the number of records or the policies of generation of the modified dataset 306), or during normal operation, in step 1404 and/or in step 1414.

In various embodiments, each constraint CS can specify that a respective variable p, for example age, is not modifiable. Additionally or alternatively, each constraint can specify, for a given variable p, for example a given laboratory value, such as cholesterol, a lower threshold and/or an upper threshold for the respective variable p. In general, the thresholds for a variable p may be predetermined (for example, constant) and/or be calculated as a function of the value of the respective variable p.

For instance, using the constraints CS, in step 1404, the computer 30 can eliminate, from the dataset 306, the variables p that are indicated as non-modifiable. Moreover, during generation of the points 304, the computer 30 can generate, in step 1406, the synthetic points 304, modifying each variable p only within the respective range of variability indicated via the respective lower threshold and/or upper threshold. Consequently, in this way, the linear classifier is trained already taking into consideration the constraints CS.

Additionally or alternatively, using the constraints CS in step 1414, the computer can inhibit evaluation of the variables that are indicated as non-modifiable and can verify whether movement of the point 300 into a class v with the lowest obtainable risk, keeping the values of the variables in the respective range of variability indicated via the respective lower threshold and/or upper threshold.

Consequently, in various embodiments, the constraints CS may define limits to the possible values of the clinical and omics variables, and/or clinical variables that cannot be altered (for example, the age of the patient, which cannot be shifted arbitrarily). Additionally or alternatively, the constraints CS may define multiple clinical variables that affect one another (for example, an increase in the times of physical activity that lowers the mean heart rate, or a drug that affects the expression of two genes at the same time). Additionally or alternatively, the constraints CS may specify pre-set profiles for the variations of one or more of the variables p. In this way, it is possible to specify overall variations of the clinical data (also comprising the omics data) p that correspond to expected variations of the clinical data p following upon given actions, such as the taking of a given drug and/or a given medical intervention and/or a given physical activity.

For instance, in various embodiments, the constraints CS may be specified in one or more of the following ways:
- one or more lists containing the definition of the range of values that are acceptable for one or more of the clinical and omics variables p; and/or
- one or more blacklists containing clinical and omics variables p that have to be discarded for development of the linear model and/or the subsequent prescription; and/or
- weighted correlation matrices that specify clinical and omics variables p that affect the clinical and omics variables originally used by the risk classifier, for example together with an estimate of the weight of their impact, possibly deriving from the literature or from other models.

In this context, the correlation matrices may also identify nonlinear relations. In fact, the dosage of a given drug could affect the heart rate in an exponential way up to a given threshold, and then stop every effect, while at the same time linearly affecting oxygen saturation.

Consequently, in various embodiments, using the constraints CS it is possible to define constraints that regard the potential variability of one or more variables p. For instance, in this way, there may be defined lists of variables p that cannot be varied, or the variation of which is practically impossible. For instance, in this way, the model can be trained in such a way as to avoid considering age as being a modifiable feature, in so far as it deems that it is not feasible, even though, from a purely mathematical standpoint, a younger patient would have better clinical prospects. Moreover, it is possible to specify actions, for example administration of drugs, medical interventions, and/or physical activities, that simultaneously affect a plurality of clinical variables p.

For example, in various embodiments, the constraints CS are taken into consideration directly during generation of the linear classifier in step 1404. For example, the previously mentioned probability distributions may be limited according to the constraints, e.g., by setting the respective variable p to a constant value (for variable which cannot be varied) or limiting the values of the respective variable p between a minimum value and a maximum value. In this way the synthetic points 304 already correspond to points of clinical data that are potentially achievable/obtainable. Consequently, by modifying the constraints CS and by training again the linear classifier in step 1404, the therapeutic options available may be updated cyclically, for example to take into account the availability of a new drug, without the need to train again the original risk classifier. In fact, as explained previously, these constraints CS do not affect the original nonlinear classifier that still uses all the original variables p (or the respective features F) for the prediction.

Conversely, in other embodiments, step 1406 generates the synthetic points as described in the foregoing without considering any constraints for the variables p, e.g., by performing the previously mentioned Monte Carlo simulation, and the constraints are taken into account when determining the vector MP. Specifically, as mentioned before, in various embodiments, each variable p may not have constraints, may be fixed to the respective value of the patient P (e.g., age) or be limited/bounded between a respective minimum and a respective maximum value (e.g., blood pressure). Accordingly, as mentioned before, in case all variables do not have constrains, the computer may calculate the vector MP as the normal to the separation plane. Conversely, in case one or more variables are fixed, a respective variable subspace may be selected, which does not comprise the fixed variable(s) p, and the computer may calculate the vector MP as the normal to the separation plane in the subspace, possibly also adding again the fixed variables to the vector MP. Finally, in case one or more variables are limited, the computer may still select for the vector MP the minimum distance between the separation plane and the point P, while satisfying the boundaries. However, in this case, the vector MP may not be perpendicular to the separation plane.

Given a new patient (considered as being represented by a set of the same clinical and omics variables used by the risk classifier), the CDSS will use the linear model previously stored (and not the original model or a static algorithm similar to a flow-chart or a bayesian-rules engine), for generating a linear classifier, the characteristics of which will enable mathematical generation of a prescription. Such a use will generate the most effective set of actions to be carried out to improve the prognosis of the patient, taking into account the constraints identified (CS), i.e., the constraints implemented in the explainer, as described previously.

In various embodiments, the computer that implements the CDSS then shows, in step 1414, to an end user the prescription in a graphic interface in a way understandable for humans. In the simplest case, the computer may display the data MP that correspond to a list of one or more clinical and omics variables p of the patient P that should be modified to change the classification of risk v of the patient P. In various embodiments, by calculating the distance between the respective dataset of modified values 304 and the boundary of separation 402, the computer may also supply an estimate of the likelihood of said prescription being effective. In the case where a list of specified actions is used via respective variations of the clinical and omics variables, the CDSS could display (in addition or as an alternative to the data MP) the name of the action, for example, indicate the name of the respective drug. For instance, for this purpose, each action may specify a modification to one or more of the clinical data p, and the computer can determine whether one of the actions is compatible with the minimum modification MP by verifying whether the direction of the modifications indicated by the respective action substantially corresponds to the direction of the modifications MP. Likewise, the computer can verify whether the degree of the modifications indicated by the respective action substantially corresponds to the degree of the modifications MP.

Consequently, the solutions described herein do not require a preliminary knowledge of the clinical domain and adapt to the needs of precision medicine, hence proposing in a proactive and personalized way the treatments for a given patient P. In fact, by definition, the prescription issued in step 1414 is specific for the patient P in so far as the same set of actions may be ineffective for a different patient.

Of course, without prejudice to the principles underlying the invention, the details of implementation and the embodiments may vary widely with respect to what has been described and illustrated herein purely to way of example, without thereby departing from the scope of the present invention, as defined by the annexed claims.

## Claims

1. A method for providing a clinical decision support, comprising executing the following steps via a computer (30):
- obtaining (1202) for a patient (P) respective values (300) of a plurality of clinical data (p);
- generating a training dataset (306) via the steps of
- estimating (1204) by means of a non-linear classifier a respective risk class (v) for the values of said plurality of clinical data (p) of said patient (P);
- adding the values of said plurality of clinical data (p) of said patient (P) and the respective estimated risk class (v) to said training dataset (306);
- generating (1406) a plurality of modified datasets (304) by modifying the values of said plurality of clinical data (p) of said patient (P);
- estimating (1408) by means of said non-linear classifier a respective risk class (v) for the values of each modified dataset (304);
- adding the values of each modified dataset (304) and the respective estimated risk class (v) to said training dataset (306);
- determining a risk class (v) among said risk classes (v) estimated for said modified datasets (304) indicating a lower risk than said risk class (v) estimated for the values of said plurality of clinical data (p) of said patient (P),
- training (1112) a linear classifier configured to estimate said risk class (v) as a function of the values of said plurality of clinical data (p) by using said training dataset (306);
- determining a separation plane (402) of said linear classifier separating the risk class (v) estimated for the values of said plurality of clinical data (p) of said patient (P) from said risk class (v) indicating a lower risk;
- using said separation plane (402) to calculate the minimum change (MP) required to the values of said plurality of clinical data (p) of said patient (P) to change the risk class (v) estimated for the values of said plurality of clinical data (p) of said patient (P) in said risk class (v) indicating a lower risk; and
- providing a support for clinical decisions by showing said change (MP) to the values of said plurality of clinical data (p) of said patient (P) on a display.

2. The method according to Claim 1, comprising the following steps during a training phase (1100):
- obtaining (1102) an additional training dataset (202) comprising for each of a plurality of reference patients (PR) respective values for said plurality of clinical data (p) and a respective value identifying a risk class (v);
- training (1112) said non-linear classifier using said additional training dataset (202), wherein said non-linear classifier is configured to estimate said risk class (v) as a function of the values of said plurality of clinical data (p).

3. The method according to Claim 1 or Claim 2, wherein said risk class (v) indicating a lower risk than said risk class (v) estimated for the values of said plurality of clinical data (p) of said patient (P) corresponds to the risk class (v) among said risk classes (v) estimated for said modified datasets (304) indicating a minimum risk.

4. The method according to Claim 3, wherein said linear classifier is a multi-class classifier, and said separation plane (402) of said linear classifier separates said risk class (v) indicating a minimum risk from all other risk classes (v) estimated for said modified datasets (304).

5. The method according to Claim 3, comprising:
- replacing the risk class (v) estimated for the values of said plurality of clinical data (p) of said patient (P) with a first value;
- associating the risk class (v) indicating a minimum risk with a second value;
- replacing each risk class (v) estimated for a respective modified dataset (304) with said first value if the respective risk class indicates a risk being greater than said risk class (v) indicating a minimum risk and said second value if the respective risk class corresponds to said risk class (v) indicating a minimum risk,
- training (1112) said linear classifier, whereby said linear classifier is a binary classifier configured to classify the values of said plurality of clinical data (p) either with said first value or said second value, wherein said separation plane (402) of said linear classifier separates said risk class (v) indicating a minimum risk from all other risk classes (v) estimated for said modified datasets (304).

6. The method according to any of the previous claims, wherein said using said separation plane (402) to calculate the minimum modification (MP) required comprises:
- determining the clinical data (p) to be modified by calculating the normal of said separation plane (402); and
- determining the extent of the modification to said clinical data (p) to be modified by calculating the minimum distance between the values of said plurality of clinical data (p) of said patient (P) and said separation plane (402).

7. The method according to any of the previous claims, comprising receiving one or more constraints (CS) for modifications to the values of said plurality of clinical data (p) of said patient (P), and at least one of:
- generating (1406) said plurality of modified datasets (304) by modifying the values of said plurality of clinical data (p) of said patient (P) as a function of said one or more constraints (CS); and/or
- verifying whether the minimum modification (MP) required to the values of said plurality of clinical data (p) of said patient (P) satisfies said one or more constraints (CS).

8. The method according to Claim 7, wherein said one or more constraints (CS) comprise at least one of:
- a blacklist containing clinical data which cannot be modified; and/or
- a list containing the definitions of the range of acceptable values for one or more of said clinical data (p); and/or
- data specifying one or more correlation matrices, wherein each correlation matrix specifies the correlation between a plurality of clinical data (p).

9. The method according to Claim 7 or Claim 8, wherein said one or more constraints (CS) comprise a list of actions, such as taking a given drug and/or a given medical intervention and/or a given physical activity, wherein each action specifies a change to one or more of said clinical data (p), and wherein said method comprises:
- determining whether one of said actions is compatible with said minimum modification (MP) required by verifying whether the direction of the changes to said one or more clinical data (p) corresponds substantially to the direction of said minimum modification (MP) required; and
- in case an action is compatible with said minimum modification (MP) required, showing said action on said display.

10. The method according to Claim 2, wherein said value identifying a risk class (v) corresponds to:
- information on whether the patient will develop a given disease;
- data that indicate the seriousness of the disease that the patient has developed;
- data that identify a disease-free survival time;
- data that identify whether a stay in hospital envisaged; or
- data that identify a risk of death, onset of complications or the likelihood of adverse events in the case of surgical operation.

11. The method according to any of the previous claims, wherein said clinical data (p) comprise one or more of: omics data, such as transcriptomics, genomics, epigenomics, metabolomics, methylomics, and/or proteomics data, data obtained via laboratory analyses, such as blood tests, radiological data, and general clinical data, such as age, sex, history of smoking.

12. A clinical decision support system, comprising a computer (30) configured to implement the method according to any of the previous claims.

13. A computer-program product that can be loaded into the memory of at least one processor and comprises portions of software code for implementing the steps of the method according to any of Claims 1 to 11.
